(19) Europäisches Patentamt
European Patent Office
Office européen des brevets

(11) Veröffentlichungsnummer: **0 525 346 A1**

# (12) EUROPÄISCHE PATENTANMELDUNG

(21) Anmeldenummer: **92109981.8**

(51) Int. Cl.5: **C07D 213/30**, A01N 43/40

(22) Anmeldetag: **13.06.92**

(30) Priorität: **26.06.91 DE 4121049**

(43) Veröffentlichungstag der Anmeldung: **03.02.93 Patentblatt 93/05**

(84) Benannte Vertragsstaaten: **AT BE CH DE DK ES FR GB GR IT LI NL PT SE**

(71) Anmelder: **BASF Aktiengesellschaft**
**Carl-Bosch-Strasse 38**
**W-6700 Ludwigshafen(DE)**

(72) Erfinder: **Zierke, Thomas, Dr.**
**Akazienstrasse 12**
**W-6737 Boehl-Iggelheim(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**

## (54) 3-Substituierte Pyridinmethanole und diese enthaltende Fungizide.

(57) Verbindungen der Formel

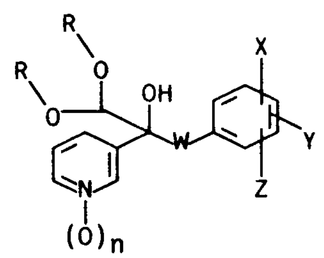

in der

| | |
|---|---|
| R | für Alkyl oder Cycloalkyl steht, |
| X,Y,Z | Wasserstoff, Halogen, Alkyl, Alkoxy, Alkoximino, Halogenalkyl, Cyano, Nitro oder gegebenenfalls substituiertes Phenyl oder Phenoxy bedeuten, |
| W | für eine Einfachbindung oder eine der Gruppen $-CH_2-$, $-CH(CH_3)-$oder $-CH_2CH_2-$ steht und |
| n | 0 oder 1 bedeutet, |

sowie deren pflanzenverträgliche Säureadditionssalze und Fungizide, die diese Verbindungen enthalten.

EP 0 525 346 A1

Die vorliegende Erfindung betrifft neue 3-Pyridinmethanole, deren Verwendung als Fungizide, fungizide Mittel, die die neuen Wirkstoffe enthalten, sowie Verfahren zur Bekämpfung von Pilzen mit diesen Wirkstoffen.

Es ist bekannt, 3-Pyridinmethanole, z.B. $\alpha,\alpha$-Bis-(4-chlorphenyl)-3-pyridinmethanol - bekannt aus US-A-3 396 224 - oder 4-(2,4-Dichlorphenyl)-3-hydroxy-3-(3-pyridyl)-2-butanon-ethylenketal - bekannt aus EP-A-209 854 - als Fungizide zu benutzen. In EP-A-435 127 werden Pyridinyl-dioxolane, z.B. das 2-(4-Fluorbenzyl)-4-(pyridin-3-yl)-5-(2,4-dichlorphenyl)-1,3-dioxolan als Fungizide beschrieben. Ihre fungizide Wirkung ist jedoch nicht immer ganz zufriedenstellend. Daraus ergibt sich die Aufgabe, neue 3-Pyridinmethanole mit anderer chemischer Struktur zu finden, die eine gute fungizide Wirkung haben.

Es wurden nun neue 3-Pyridylmethanole der Formel 1

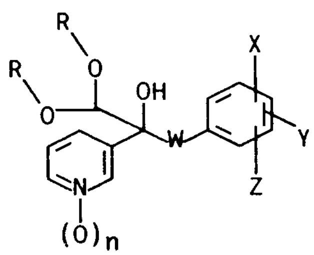

1,

in der

| | |
|---|---|
| R | für geradkettiges oder verzweigtes $C_1$-$C_6$-Alkyl oder für $C_3$-$C_6$-Cycloalkyl steht, |
| X,Y,Z | unabhängig voneinander Wasserstoff, Halogen, $C_1$-$C_4$-Alkyl, $C_1$-$C_4$-Alkoxy, $C_1$-$C_4$-Alkoximino, Halogenalkyl, Cyano, Nitro oder gegebenenfalls durch Halogen substituiertes Phenyl oder Phenoxy bedeuten, |
| W | für eine Einfachbindung oder eine der Gruppen $-CH_2-$, $-CH(CH_3)-$ oder $-CH_2CH_2-$ steht und |
| n | 0 oder 1 bedeutet, |

sowie deren pflanzenverträgliche Säureadditionssalze gefunden. Die neuen Verbindungen zeigen eine überraschend gute fungizide Wirkung, die besser ist als die Wirkung bekannter Fungizide.

R bedeutet bsw. $C_1$-$C_4$-Alkyl, insbesondere Methyl, Ethyl, n-Propyl, iso-Propyl, n-Butyl, But-2-yl, iso-Butyl, tert.-Butyl, n-Pentyl, Pent-2-yl, Pent-3-yl, neo-Pentyl, 3-Methylbut-2-yl, n-Hexyl, Hex-2-yl, Hex-3-yl, 2-Methylpent-1-yl, 3-Methylpent-1-yl, 4-Methylpent-1-yl, 2-Ethylbut-1-yl, 3-Ethylbut-1-yl, Cyclopropyl, Cyclobutyl, Cyclopentyl, Cyclohexyl.

Durch die Substituenten X, Y und Z und den Phenylring werden bis zu dreifach substituierte Phenylringe von der Erfindung umfaßt, wie z.B. Phenyl, Biphenyl, Biphen-2-yl, Biphen-3-yl, Biphen-4-yl, Halogenphenyl, 4-Chlorphenyl, 4-Fluorphenyl, 4-Bromphenyl, 2-Chlorphenyl, 2-Fluorphenyl, 2-Bromphenyl, 2,4-Dichlorphenyl, 2-Brom-4-chlorphenyl, 2-Chlor-4-bromphenyl, 2-Chlor-4-fluorphenyl, 2,3-Dichlorphenyl, 2-Chlor-3-fluorphenyl, 2-Fluor-4-chlorphenyl, 2-Chlor-3-fluor-4-chlorphenyl, 2-Brom-4-fluorphenyl, 2-Brom-3-fluor-4-chlorphenyl, 2,6-Dichlorphenyl, Halogen-$C_1$-$C_4$-alkylphenyl, 2-Trifluormethylphenyl, 3-Trifluormethylphenyl, 4-Trifluormethylphenyl, 2-Chlor-4-trifluormethylphenyl, 2-Chlor-3-trifluormethylphenyl, 2,3-Dichlor-4-trifluormethylphenyl, 2-Chlor-4-phenyl-phenyl, $C_1$-$C_4$-Alkylphenyl, 2-Methylphenyl, 4-Methylphenyl, 4-tert.-Butylphenyl, 2-Chlor-4-methylphenyl, 2-Methyl-4-chlorphenyl, 2,4-Dimethylphenyl, 2,3-Dimethylphenyl, $C_1$-$C_4$-Alkoxyphenyl, 2-Methoxyphenyl, 4-Methoxyphenyl, 2-Chlor-4-methoxyphenyl, 2-Methyl-4-methoxyphenyl, 4-tert.-Butoxyphenyl, $C_1$-$C_4$-Alkoximinophenyl, 4-Methoximinophenyl, 4-Ethoximinophenyl, 2-Chlor-4-methoximinophenyl, 4-Cyanophenyl, 2-Chlor-4-cyanophenyl, 2-Cyano-4-chlorphenyl, 2-Cyanophenyl, 2-Nitrophenyl, 4-Nitrophenyl, 2-Chlor-4-nitrophenyl, 4-Phenoxyphenyl, 4-(4'-Chlorphenoxy)phenyl.

Für Pflanzen verträgliche Salze sind z.B. Säureadditionssalze mit anorganischen Mineralsäuren wie Hydrochloride, Hydrobromide, Sulfate, Phosphate, Nitrate, Salze mit Ameisensäure oder mit Alkylcarbonsäuren wie Acetate, 2-Ethylhexanoate, Oxalate, Salze mit Arylsulfonsäuren wie Benzolsulfonate, Toluolsulfonate, Dodecylbenzolsulfonate.

Die neuen Verbindungen der Formel 1 enthalten im allgemeinen Asymmetriezentren. Sie werden im allgemeinen als Racemate oder gegebenenfalls als Diastereomerengemische erhalten. Diastereomerenreine Verbindungen lassen sich bei einigen der neuen Verbindungen durch Destillation, Säulenchromatographie oder aufgrund von Löslichkeitsunterschieden isolieren. Enantiomerenreine Verbindungen lassen sich z.B. durch Racematspaltung mit chiralen Hilfsreagenzien nach bekannten Methoden bsw. über diastereomere Salze erhalten. Für die Anwendung der neuen Verbindungen als Fungizide sind sowohl die Diastereomeren bzw. die Enantiomeren als auch die bei der Synthese anfallenden Stereoisomerengemische geeignet. Sie

alle werden von der Erfindung umfaßt.

Verbindungen der Formel 1, in denen n = 1 bedeutet, werden aus den entsprechenden Verbindungen der Formel 1 mit n = 0 durch Behandlung mit einer Persäure oder mit Gemischen aus Säuren, wie Essigsäure und Waserstoffperoxid erhalten.

Die Herstellungsverfahren für Verbindungen der Formel 1, in denen n = 0 ist, lassen sich durch folgende Reaktionsgleichungen veranschaulichen:

Gleichung 1:

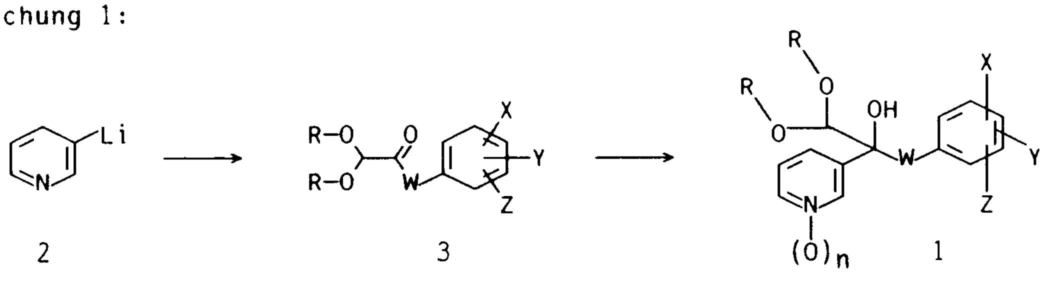

2 3 1

Gleichung 2:

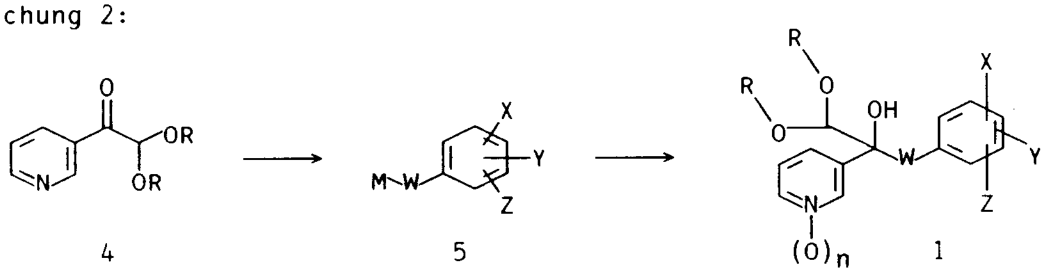

4 5 1

In den Formeln 2, 4 und 5 haben W, X, Y, Z und R die gleiche Bedeutung wie in Formel 1. M steht in Formel 5 z.B. für Lithium oder die Reste MgCl oder MgBr. Reaktionen mit metallorganischen Agenzien, wie durch Gl. 1 und 2 veranschaulicht, sind allgemein bekannte Reaktionen der organischen Chemie (siehe z.B. Organikum, 15. Aufl. 1977, S. 623ff). Zweckmäßig wird eine solche Reaktion so durchgeführt, daß man 1 bis 2 Äquivalente der Organometallverbindung in einem inerten Lösungsmittel, vorzugsweise Diethylether, Methyl-tert.-butylether, Tetrahydrofuran oder einem Gemisch derselben, vorlegt und die Ketonkomponente bei -50 °C bis +50 °C zudosiert. Ketone der Formel 3 sind allgemein bekannte Verbindungen, zum Teil aber auch neue Verbindungen. Sie lassen sich durch eine Vielzahl von bekannten Methoden herstellen, wie sie z.B. in EP-A-222 217 oder in DE-A-40 26 788 beschrieben werden. Eine ebenfalls bevorzugte Methode wird für den Fall, daß R = $CH_3$, W = $CH_2$ und M = MgCl bedeutet, durch Gl. 3 veranschaulicht.

Gleichung 3:

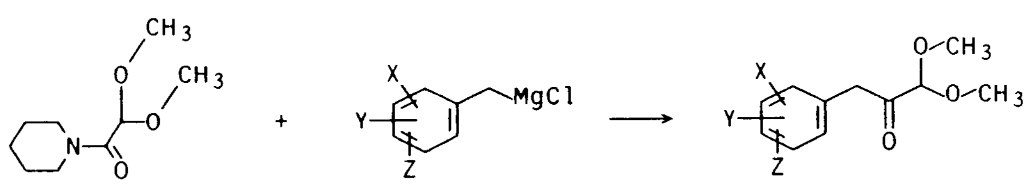

6 7 8

Verbindung 6 ist bekannt (K. Schank, Chem.Ber. 100, S. 2292-5, 1967). Sie dient, wie ebenfalls in der Literatur beschrieben (Baldwin et al., J.Med. Chem. 22, S. 692, 1979), auch als Ausgangsprodukt für Verbindung 4, wenn R = $CH_3$ bedeutet. Die Darstellung von 3-Pyridyllithium 2 erfolgt wie in der Literatur beschrieben (W.E. Parham et al, JOC 42 1977, S. 257f).

Die folgenden Beispiele beschreiben die Herstellung der neuen Verbindungen:

Beispiel 1

Herstellung von 1, 1-Dimethoxy-2-hydroxy-2-(3-pyridyl)-3-(2,4-dichlorphenyl)-propan (Beispiel 4 der Tabelle)

Zu einer Lösung von 0,371 mol 3-Pyridyllithium in 1,1 l Diethylether ($Et_2O$), hergestellt aus n-Butyllithium und 3-Brompyridin, werden bei-70°C 88,9 g (0,337 mol) 1,1-Dimethoxy-3-(2,4-dichlorphenyl)-propan-2-on in 100 ml $Et_2O$ zugetropft. Nach einer halben Stunde bei -70°C wird langsam auf 0°C aufgewärmt. Nach Zugabe von iso-Propanol und anschließend Wasser wird die Reaktionslösung mit 1 n Salzsäure extrahiert. Die saure wäßrige Phase wird anschließend mit $NaHCO_3$ neutralgestellt und mit Methylenchlorid ($CH_2Cl_2$) extrahiert. Nach Trocknen über $Na_2SO_4$ werden alle flüchtigen Bestandteile am Rotationsverdampfer abgezogen. Man erhält ein gelbrotes Öl.
Ausbeute: 56,1 g (48,7 %)
1H-NMR ($CDCl_3/TMS_{int}$): δ/ppm = 3.08 (s,breit, 1H), 3,33 (d, 1H), 3,48 (d, 1H), 3,53 (s, 6H), 4,35 (s, 1H), 6,98 (d, 2H), 7,20 (dd, 1H), 7,28 (d, 1H), 7,75 (m, 1H), 8,5 (dd, 1H), 8,73 (d, 1H)

Vorschrift 1

Herstellung des Edukts 1, 1-Dimethoxy-3-(2,4-dichlorphenyl)-propan-2-on

Zu einer Lösung von 2,4-Dichlorbenzylmagnesiumchlorid in 300 ml Diethylether ($Et_2O$), hergestellt aus 13,2 g (0,55 mol) Mg-Spänen und 76,8 g (0,55 mol) 2,4-Dichlorbenzylchlorid, werden 95 g (0,5 mol) Dimethoxyessigsäurepiperidid, gelöst in 200 ml Tetrahydrofuran (THF), getropft. Nach 2 Stunden bei Raumtemperatur (20°C) und 2 Stunden bei 50°C wird durch Zugabe von 250 ml kalter 20 %iger $NH_4Cl$-Lösung aufgearbeitet. Die wäßrige Phase wird noch zweimal mit $Et_2O$ extrahiert. Die vereinigten organischen Phasen werden über $Na_2SO_4$ getrocknet, abfiltriert und eingeengt. Man erhält ein gelbes Öl.
Ausbeute: 73 g (56 %)
$^1$H-NMR ($CDCl_3/TMS_{int}$): δ/ppm = 3.48 (s, 6H), 4,0 (s, 2H), 4,55 (s, 1H), 3,2 (d, 1H), 3,4 (d, 1H), 7,08-7,4 (m,3H)
In entsprechender Weise lassen sich die folgenden erfindungsgemäßen Verbindungen der allgemeinen Formel 1

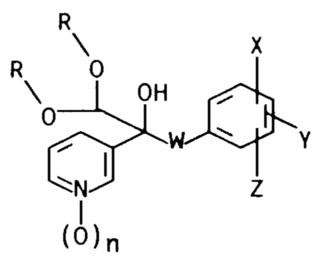

in der n = 0 bedeutet, erhalten.

Tabelle

| Nr. | W | R | 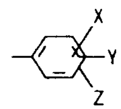 | Physik. Daten |
|---|---|---|---|---|
| 1 | - | Methyl | 4-Fluorphenyl | Fp.: 105-110°C |
| 2 | $-CH_2-$ | Methyl | Phenyl | Öl, NMR: 3,5(s,6H) |
| 3 | $-CH_2-$ | Methyl | 2,4-Dimethylphenyl | Öl, NMR: 2,1(s,3H), 2,23(s,3H), 3,5(s,3H) |
| 4 | $-CH_2-$ | Methyl | 2,4-Dichlorphenyl | Öl, NMR: 3,53(s,6H) |
| 5 | $-CH_2-$ | Methyl | 4-Chlorphenyl | Öl, NMR: 3,2(d,2H), 3,5(s,6H), 4,3(s,1H) |
| 6 | - | Methyl | Phenyl | Öl, NMR: 3,38(s,3H), 3,4(s,3H), 4,7(s,1H) |
| 7 | - | Methyl | 2,4-Dichlorphenyl | Öl, NMR: 3,3(s,3H), 3,6(s,3H), 5,1(s,1H) |
| 8 | - | Methyl | 4-Methylphenyl | Öl, NMR: 2,33(s,3H), 3,38(s,3H), 3,42(s,3H), 4,7(s,1H) |
| 9 | - | Methyl | 4-Methoxyphenyl | Öl, NMR: 3,38(s,3H), 3,42(s,3H), 3,8(s,3H), 4,65(s,1H) |
| 10 | - | Methyl | Biphen-4-yl | Öl, NMR: 3,4(s,3H), 3,42(s,3H), 4,7(s,1H) |
| 11 | $-CH_2-$ | Methyl | 2-Chlorphenyl | Öl, NMR: 3,35(d,1H), 3,52(s,6H), 3,56(d,1H), 4,4(s,1H) |
| 12 | $-CH_2-$ | Methyl | 3,4-Dichlorphenyl | Öl, NMR: 3,13(d,1H), 3,2(d,1H), 3,50(s,3H), 3,52(s,3H), 4,3(s,1H) |
| 13 | $-CH_2-$ | Methyl | 3-Trifluorphenyl | Fp.: 94-103°C |
| 14 | - | Methyl | 4-Chlorphenyl | Öl, NMR: 3,4(s,6H), 4,6(s,1H) |
| 15 | $-CH_2-$ | Methyl | 4-Fluorphenyl | Öl, NMR: 3,2(dd,2H), 3,5(s,6H), 4,32(s,1H) |
| 16 | $-CH_2-$ | Methyl | 2-Chlor-6-fluorphenyl | Fp.: 63-65°C |
| 17 | - | Ethyl | 4-Fluorphenyl | |
| 18 | $-CH_2-$ | Ethyl | Phenyl | |
| 19 | $-CH_2-$ | Ethyl | 2,4-Dimethylphenyl | |

Tabelle (Fortsetzung)

| Nr. | W | R | 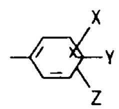 | Physik. Daten |
|---|---|---|---|---|
| 20 | $-CH_2-$ | Ethyl | 2,4-Dichlorphenyl | |
| 21 | $-CH_2-$ | Ethyl | 4-Chlorphenyl | |
| 22 | - | Ethyl | Phenyl | |
| 23 | - | Ethyl | 2,4-Dichlorphenyl | |
| 24 | - | Ethyl | 4-Methylphenyl | |
| 25 | - | Ethyl | 4-Methoxyphenyl | |
| 26 | - | Ethyl | Biphen-4-yl | |
| 27 | $-CH_2-$ | Ethyl | 2-Chlorphenyl | |
| 28 | $-CH_2-$ | Ethyl | 3,4-Dichlorphenyl | |
| 29 | $-CH_2-$ | Ethyl | 3-Trifluorphenyl | |
| 30 | - | Ethyl | 4-Chlorphenyl | |
| 31 | $-CH_2-$ | Ethyl | 4-Fluorphenyl | |
| 32 | $-CH_2-$ | Ethyl | 2-Chlor-6-fluorphenyl | |
| 33 | - | n-Propyl | 4-Fluorphenyl | |
| 34 | $-CH_2-$ | n-Propyl | Phenyl | |
| 35 | $-CH_2-$ | n-Propyl | 2,4-Dimethylphenyl | |
| 36 | $-CH_2-$ | n-Propyl | 2,4-Dichlorphenyl | |
| 37 | $-CH_2-$ | n-Propyl | 4-Chlorphenyl | |
| 38 | - | n-Propyl | Phenyl | |

Tabelle (Fortsetzung)

| Nr. | W | R | 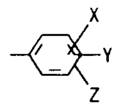 | Physik. Daten |
|---|---|---|---|---|
| 39 | - | n-Propyl | 2,4-Dichlorphenyl | |
| 40 | - | n-Propyl | 4-Methylphenyl | |
| 41 | - | n-Propyl | 4-Methoxyphenyl | |
| 42 | - | n-Propyl | Biphen-4-yl | |
| 43 | $-CH_2-$ | n-Propyl | 2-Chlorphenyl | |
| 44 | $-CH_2-$ | n-Propyl | 3,4-Dichlorphenyl | |
| 45 | $-CH_2-$ | n-Propyl | 3-Trifluorphenyl | |
| 46 | - | n-Propyl | 4-Chlorphenyl | |
| 47 | $-CH_2-$ | n-Propyl | 4-Fluorphenyl | |
| 48 | $-CH_2-$ | n-Propyl | 2-Chlor-6-fluorphenyl | |
| 49 | - | n-Butyl | 4-Fluorphenyl | |
| 50 | $-CH_2-$ | n-Butyl | Phenyl | |
| 51 | $-CH_2-$ | n-Butyl | 2,4-Dimethylphenyl | |
| 52 | $-CH_2-$ | n-Butyl | 2,4-Dichlorphenyl | |
| 53 | $-CH_2-$ | n-Butyl | 4-Chlorphenyl | |
| 54 | - | n-Butyl | Phenyl | |
| 55 | - | n-Butyl | 2,4-Dichlorphenyl | |
| 56 | - | n-Butyl | 4-Methylphenyl | |
| 57 | - | n-Butyl | 4-Methoxyphenyl | |

Tabelle (Fortsetzung)

| Nr. | W | R | 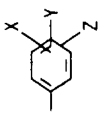 | Physik. Daten |
|---|---|---|---|---|
| 58 | - | n-Butyl | Biphen-4-yl | |
| 59 | $-CH_2-$ | n-Butyl | 2-Chlorphenyl | |
| 60 | $-CH_2-$ | n-Butyl | 3,4-Dichlorphenyl | |
| 61 | $-CH_2-$ | n-Butyl | 3-Trifluorphenyl | |
| 62 | - | n-Butyl | 4-Chlorphenyl | |
| 63 | $-CH_2-$ | n-Butyl | 4-Fluorphenyl | |
| 64 | $-CH_2-$ | n-Butyl | 2-Chlor-6-fluorphenyl | |
| 65 | $-CH_2-$ | iso-Propyl | 2,4-Dichlorphenyl | |
| 66 | $-CH_2-$ | Methyl | 2-Fluor-4-chlorphenyl | |
| 67 | $-CH_2-$ | Methyl | 4-Fluor-2-chlorphenyl | |
| 68 | $-CH_2-$ | c-Hexyl | 2,4-Dichlorphenyl | |
| 69 | $-CH_2-$ | Ethyl | 2-Fluor-4-chlorphenyl | |
| 70 | $-CH_2-$ | Ethyl | 4-Fluor-2-chlorphenyl | |

Verbindungen der Formel 1, in denen n = 1 bedeutet, lassen sich aus den Verbindungen der Tabelle herstellen, indem man diese Verbindungen mit einer Persäure wie meta-Chlorperbenzoesäure oder Peressigsäure behandelt und anschließend durch eine Wäsche mit $NaHCO_3$-Lösung die entstandene Carbonsäure abtrennt.

Die neuen Verbindungen der Formel 1 eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung Nr. 1 und 10 Gew.-Teilen N-Methyl-$\alpha$-pyrrolidon, die zur Anwendung in Form kleinster Tropfen geeignet ist;

II. eine Mischung aus 20 Gew.-Teilen der Verbindung Nr. 2, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 3, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 4, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 5, 3 Gew.-Teilen des Natriumsalzes der Diisobutylnaphtalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 6 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 7, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 8, 10 Gew.-Teilen des Natriumsalzes eines Phenosulfonsäureharnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 9, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
Ferridimethyldithiocarbamat,
Zinkdimethyldithiocarbamat,

Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,

Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3--chlor-2-aminopyridin,
1-((bis-(4-Fluorphenyl)-methylsilyl)-methyl)-1H-1,2,4-triazol.

Anwendungsbeispiele

Als Vergleichswirkstoff wurde 2-(4-Fluorbenzyl)-4-(pyridin-3-yl)-5-(2,4-dichlorphenyl)-1,3-dioxolan (A) - beschrieben in EP 435 127 - benutzt.

Anwendungsbeispiel 1

Wirksamkeit gegen Botrytis cinerea

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedecken.

Das Ergebnis zeigt, daß die Wirkstoffe 3, 4, 5, 10, 11, 12 und 13 bei der Anwendung als 500 ppm Wirkstoff enthaltende Spritzbrühe eine bessere fungizide Wirkung zeigen (10 % Befall der Blätter) als der bekannte Vergleichswirkstoff A (70 % Befall der Blätter).

Anwendungsbeispiel 2

Wirksamkeit gegen Pyrenophora teres

Gerstenkeimlinge der Sorte "Igri" wurden im Zweikeimblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18 °C gestellt. Anschließend wurden die Pflanzen im Gewächshaus bei 20 bis 22 °C und 70 % relativer Luftfeuchtigkeit für weitere 5 Tage kultiviert. Dann wurde das Ausmaß des Blattbefalls ermittelt.

Das Ergebnis zeigt, daß die Wirkstoffe 3, 4 und 5 bei der Anwendung als 500 ppm Wirkstoff enthaltende Spritzbrühe eine bessere fungizide Wirkung zeigen (10 % Befall der Blätter) als der bekannte Vergleichswirkstoff A (50 % Befall der Blätter).

**Patentansprüche**

**1.** Verbindungen der Formel 1

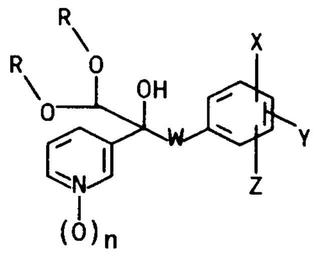

1,

in der

R für $C_1$-$C_6$-Alkyl oder $C_3$-$C_6$-Cycloalkyl steht,

X,Y,Z unabhängig voneinander Wasserstoff, Halogen, $C_1$-$C_4$-Alkyl, $C_1$-$C_4$-Alkoxy, $C_1$-$C_4$-Alkoximino, Halogenalkyl, Cyano, Nitro oder gegebenenfalls durch Halogen substituiertes Phenyl oder Phenoxy bedeuten,

W für eine Einfachbindung oder eine der Gruppen $-CH_2-$, $-CH(CH_3)-$ oder $-CH_2CH_2-$ steht und

n 0 oder 1 bedeutet,

sowie deren pflanzenverträgliche Säureadditionssalze.

**2.** Fungizid, enthaltend einen festen oder flüssigen Trägerstoff und eine fungizid wirksame Menge einer Verbindung der Formel 1

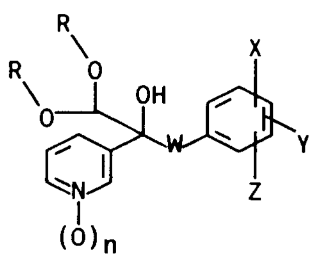

1,

in der

R für $C_1$-$C_6$-Alkyl oder $C_3$-$C_6$-Cycloalkyl steht,

X,Y,Z unabhängig voneinander Wasserstoff, Halogen, $C_1$-$C_4$-Alkyl, $C_1$-$C_4$-Alkoxy, $C_1$-$C_4$-Alkoximino, Halogenalkyl, Cyano, Nitro oder gegebenenfalls durch Halogen substituiertes Phenyl oder Phenoxy bedeuten,

W für eine Einfachbindung oder eine der Gruppen $-CH_2-$, $-CH(CH_3)-$ oder $-CH_2CH_2-$ steht und

n 0 oder 1 bedeutet,

oder deren pflanzenverträgliche Säureadditionssalze.

**3.** Verfahren zur Bekämpfung von Pilzen, dadurch gekennzeichnet, daß man eine fungizid wirksame Menge einer Verbindung der Formel 1

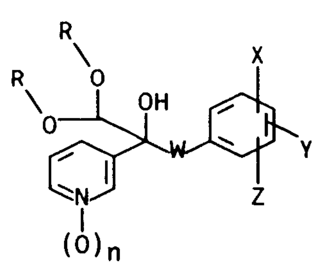

1,

in der

R für $C_1$-$C_6$-Alkyl oder $C_3$-$C_6$-Cycloalkyl steht,

X,Y,Z unabhängig voneinander Wasserstoff, Halogen, $C_1$-$C_4$-Alkyl, $C_1$-$C_4$-Alkoxy, $C_1$-$C_4$-Alkoximino, Halogenalkyl, Cyano, Nitro oder gegebenenfalls durch Halogen substituiertes

Phenyl oder Phenoxy bedeuten,

W für eine Einfachbindung oder eine der Gruppen $-CH_2-$, $-CH(CH_3)-$ oder $-CH_2CH_2-$ steht und

n 0 oder 1 bedeutet,

oder deren pflanzenverträgliche Säureadditionssalze auf die Pilze oder die vor Pilzbefall zu schützenden Pflanzen, Saatgüter, Materialien oder den Erdboden einwirken läßt.

**4.** Verfahren zur Herstellung einer Verbindung der Formel 1

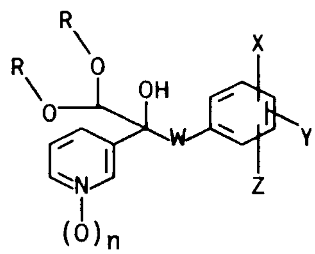

1,

in der

R für $C_1$-$C_6$-Alkyl oder $C_3$-$C_6$-Cycloalkyl steht,

X,Y,Z unabhängig voneinander Wasserstoff, Halogen, $C_1$-$C_4$-Alkyl, $C_1$-$C_4$-Alkoxy, $C_1$-$C_4$-Alkoximino, Halogenalkyl, Cyano, Nitro oder gegebenenfalls durch Halogen substituiertes Phenyl oder Phenoxy bedeuten,

W für eine Einfachbindung oder eine der Gruppen $-CH_2-$, $-CH(CH_3)-$ oder $-CH_2CH_2-$ steht und

n 0 oder 1 bedeutet,

dadurch gekennzeichnet, daß man

a) eine Verbindung der Formel

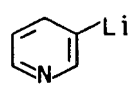

2

mit einer Verbindung der Formel

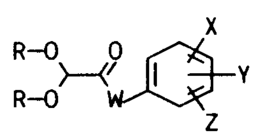

3

umsetzt oder

b) eine Verbindung der Formel

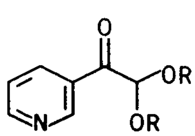

4

mit einer Verbindung der Formel

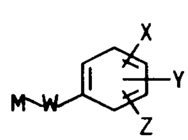

5

umsetzt, wobei W, X, Y, Z und R die obengenannten Bedeutungen haben und M Li oder MgCl oder MgBr bedeutet.

**5.** Verbindung der Formel 1 gemäß Anspruch 1, in der W eine Einfachbindung, R Methyl und X und Y H und Z 4-Fluor bedeutet.

**6.** Verbindung der Formel 1 gemäß Anspruch 1, in der W $-CH_2-$, R Methyl und X, Y, Z H bedeuten.

**7.** Verbindung der Formel 1 gemäß Anspruch 1, in der W $-CH_2-$, R Methyl und X H, Y 2-Methyl und Z 4-Methyl bedeuten.

**8.** Verbindung der Formel 1 gemäß Anspruch 1, in der W $-CH_2-$, R Methyl und X H, Y 2-Chlor und Z 4-Chlor bedeuten.

Europäisches Patentamt

# EUROPÄISCHER RECHERCHENBERICHT

Nummer der Anmeldung

EP 92 10 9981

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D,<br>P | EP-A-0 435 127 (BASF AKTIENGESELLSCHAFT)<br>* Ansprüche; Beispiele 7-10; Tabellen 4,5 *<br>--- | 1-8 | C07D213/30<br>A01N43/40 |
| A,D | EP-A-0 209 854 (F.HOFFMANN-LA ROCHE & CO. AKTIENGESELLSCHAFT)<br>* Ansprüche 1-21; Beispiel 9 *<br>--- | 1-8 | |
| A | EP-A-0 400 344 (BASF AKTIENGESELLSCHAFT)<br>* das ganze Dokument *<br>----- | 1-8 | |
| | | | RECHERCHIERTE SACHGEBIETE (Int. Cl.5 ) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 22 SEPTEMBER 1992 | P. BOSMA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)